# EUROPEAN PATENT SPECIFICATION

(11) **EP 3 524 153 B1**
(45) Date of publication and mention of the grant of the patent: **27.09.2023**
(21) Application number: 17858613.7
(22) Date of filing: 30.08.2017
(51) Int. Cl.: A61B 5/151, G01N 33/487

(54) **CARTRIDGE TYPE BIOINFORMATION MEASUREMENT DEVICE**
KASSETTENARTIGE VORRICHTUNG ZUR MESSUNG VON BIOLOGISCHEN INFORMATIONEN
DISPOSITIF DE MESURE DE BIO-INFORMATIONS DE TYPE CARTOUCHE

(30) Priority: 06.10.2016 KR 20160129233
(43) Date of publication of application: 14.08.2019
(73) Proprietor: I-sens, Inc., Seoul 06646 (KR)
(72) Inventor: LEE, Jin Won, Seoul 04724 (KR); CHOI, Hyun Ho, Seoul 03097 (KR); CHA, Geun Sig, Seoul 03610 (KR); NAM, Hak Hyun, Seoul 01227 (KR)
(74) Representative: Sander, Rolf
(86) International application number: PCT/KR2017/009506
(87) International publication number: WO 2018/066818

(56) References cited:
- WO-A1-2013/069223
- WO-A2-02/078533
- JP-A- 2007 187 674
- JP-B2- 4 566 231
- KR-A- 20090 093 696
- KR-A- 20090 093 696
- KR-A- 20150 061 100
- KR-A- 20150 129 252
- US-A1- 2012 305 419

## Description

### TECHNICAL FIELD

The present disclosure relates to a cartridge type bioinformation measurement device, and more specifically, to a cartridge type bioinformation measurement device, in which in the cartridge type bioinformation measurement device including a cartridge and a measurement body on which the cartridge is detachably mounted, it is possible to accurately confirm a remaining number of sensor strips in the cartridge by autonomously detecting whether a sensor strip has been used, even when the cartridge used to store and manage the remaining number of sensor strips therein is mounted on another measurement body, it is possible to easily confirm the number of the sensor strips remaining in the cartridge, and it is possible to display the number of the sensor strips remaining in the cartridge on a display unit of the measurement body when the cartridge and the measurement body are coupled by determining whether the cartridge and the measurement body have been coupled.

### BACKGROUND ART

Generally, a bio-measurer, such as a blood glucose meter, is a device used to measure the health condition of a user by measuring biometric values of the user, such as the level of blood glucose. In general, biometric values of a user are measured by applying an object to be measured on a testing strip, i.e., a sensor strip. For example, in the case of a blood glucose meter, a sensor strip of the blood glucose meter is fitted into a slit, and a small amount of blood is gathered from the tip of a user's finger. When the gathered blood is brought into contact with the sensor strip of the blood glucose meter, the blood is automatically absorbed into the slit of the sensor strip, and a measured value of blood glucose is then displayed on a display. A measurement device measures bioinformation using a sensor strip in the form of a strip, and the sensor strip should be replaced after the bioinformation is measured.

Recently, a cartridge type product, in which a plurality of sensor strips are accommodated in a single case, so as to increase user convenience, has appeared. Such a bioinformation measurement device, into which a sensor strip cartridge is inserted, may reduce the inconvenience of replacing the sensor strip whenever a user measures any bioinformation.

Korean Patent No. 10-1348410 discloses "STRIP ACCOMMODATION DEVICE FOR MEASURING BLOOD GLUCOSE AND STRIP STORAGE DEVICE." Specifically, the strip accommodation device includes a housing in which a plurality of sensor strips aligned in a row to collect a body fluid are stored, an operation member which has one side open such that the housing is inserted thereinto in order to discharge the strip and is rotated at a certain angle with respect to a rotation shaft installed on an outer surface of the housing, a first transfer member installed in the housing so as to discharge the sensor strip through an outlet and converts the rotational motion of the operation member into an upward rectilinear motion to transfer the strip upwardly, and a second transfer member which has one side installed in a position opposite to the outlet in the housing and transfers an unused sensor strip to an outlet region.

According to the sensor strip accommodation device of Korean Patent No. 10-1348410, one of the plurality of sensor strips is automatically discharged through the outlet, thereby reducing a discharge time of the sensor strip and facilitating the portability and operability of a product.

Thorough gluco-regulation is the initial basis of diabetes management including the prevention of complications. Therefore, a user must regularly check for changes in glucose levels, which may change frequently during the day. The user should use a sensor strip whenever measuring the level of the glucose, so that the user should take care not to run out of sensor strips. However, in the case of a cartridge in which a plurality of sensor strips are provided in an inner space, it may be difficult for the user to confirm the number of sensor strips remaining in the cartridge. There is an inconvenience in that,to purchase sensor strips when the number of the sensor strips is nearing its limit, the user should directly note or remember the number of remaining sensor strips.

KR20090093696A discloses that a method and a system for managing test sheet for measuring blood sugar are provided to monitor total stock amount of wasted product and automatically deliver the test sheet to a user. A method for managing a test sheet for measuring blood sugar comprises: a step of setting accumulated value of counter machine using the number of test sheet for measuring blood sugar; a step of replying to event signal generated and renewing the accumulated value; and a step of providing information related to the supplement of the test sheet in case that the newly accumulated value corresponds to n value.

JP2007187674A also discloses a cartridge type bioinformation measurement device with management means to determine the residual amount of biosensor test strips.

### DISCLOSURE

### Technical Problem

Accordingly, the present invention has been made in consideration of the above-described problems occurring in a cartridge type bioinformation measurement device according to the related art, and the present invention provides a cartridge type bioinformation measurement device capable of providing a notification of a number of sensor strips remaining in an interior space of a cartridge.

The present invention also provides a cartridge type bioinformation measurement device, in which in the cartridge type bioinformation measurement device, including a cartridge and a measurement body on which the cartridge is detachably mounted, since it is autonomously detected whether a sensor strip has been used, a remaining number of sensor strips is stored in the cartridge, and even when a used cartridge is mounted on another measurement body, it is possible to easily confirm the number of the sensor strips remaining in the cartridge.

The present invention also provides a cartridge type bioinformation measurement device, in which a remaining number of sensor strips is displayed on a display unit of the measurement body when a cartridge and a measurement body are coupled by determining whether the cartridge and the measurement body have been coupled.

The present invention also provides a cartridge type bioinformation measurement device, in which a number of sensor strips remaining in a cartridge is compared with a set remaining number, and when the number of remaining sensor strips is less than the set remaining number, an order message requesting an order for a new cartridge is generated and transmitted to a set user terminal, whereby cartridges can be easily ordered.

The present invention also provides a cartridge type bioinformation measurement device, in which a sensor strip is exposed and disposed in an outlet, and an optical sensor is disposed in the outlet to detect that the sensor strip is discharged through the outlet again, thereby accurately determining whether the sensor strip has been used.

### Technical Solution

In accordance with the present invention, there is provided a cartridge type bioinformation measurement device as defined by claim 1. Further advantageous embodiments of the present invention are defined by the dependent claims. According to the present disclosure, a cartridge type bioinformation measurement device includes a cartridge having an interior space in which a plurality of strips for measuring bioinformation are stacked; and a measurement body on which the cartridge is detachably mounted, the measurement body measuring the bioinformation through the strip exposed from the cartridge, wherein terminals are formed on one side of the cartridge and the other side of the measurement body facing the one side and come into contact with each other when the cartridge is mounted on the measurement body, and a management module is disposed in the cartridge to count and store a remaining number of the strips and provide information on the remaining number of the strips to the measurement body through the terminals.

The management module includes a counter counting the remaining number of the strips remaining in the interior space based on whether the strip has been used; a storage storing the remaining number of the strips; and a provider providing the information on the remaining number of the strips to the measurement body through the terminals.

The management module includes a detector detecting whether the strip has been used by determining whether the strip has been exposed from the interior space in order to measure bioinformation or determining whether the strip has been discharged from an exposure mounting position after the bioinformation has been measured.

Here, the detector is an optical sensor and may detect whether the strip is exposed or detects whether the strip is discharged from the exposure mounting position.

Preferably, the measurement body according to the embodiment of the present disclosure may further include a display unit displaying the information on the remaining number of the strips received from the management module.

Here, the management module may determine whether the terminal formed on the one side of the cartridge and the cartridge formed at the other side of the measurement body come into contact with each other, and when the terminal on the one side and the other terminal at the other side come into contact with each other, the management module may provide the information on the remaining number of the strips to the measurement body and displays the information on the remaining number on the display unit.

Preferably, the measurement body according to the embodiment of the present disclosure may further include an order manager generating an order message for requesting an order for a new cartridge and transmits the order message to a set user terminal.

Preferably, the measurement body may further include a comparator comparing the remaining number of the sensor strips received from the management module with a preset critical remaining number, and when the remaining number of the sensor strips is lower than the critical remaining number, the order manager may generate the order message.

### Advantageous Effects

As set forth above, a cartridge type bioinformation measurement device according to the present disclosure has various effects as follows.

First, a cartridge type bioinformation measurement device according to the present disclosure may notify a user of a number of sensor strips remaining in an interior space of a cartridge by detecting whether the sensor strip of the cartridge has been used.

Second, in a cartridge type bioinformation measurement device according to the present disclosure, which includes a cartridge and a measurement body on which the cartridge is detachably coupled, since it is autonomously detected whether a sensor strip has been used in the cartridge and a remaining number of sensor strips is stored in the cartridge, even when a used cartridge is mounted on another measurement body, it is possible to easily confirm the number of the sensor strips remaining in the cartridge.

Third, in a cartridge type bioinformation measurement device according to the present disclosure, a remaining number of sensor strips is displayed on a display unit of the measurement body when a cartridge and a measurement body are coupled by determining whether the cartridge and the measurement body have been coupled.

Fourth, in a cartridge type bioinformation measurement device according to the present disclosure, a number of sensor strips remaining in a cartridge may be compared with a critical remaining number, and when the number of remaining sensor strips is lower than the critical remaining number, an order message requesting an order for a new cartridge may be generated and transmitted to a set user terminal, thereby easily ordering the cartridge.

Fifth, in a cartridge type bioinformation measurement device according to the present disclosure, a sensor strip may be exposed and disposed in an outlet, and an optical sensor may be disposed in the outlet to detect that the sensor strip is discharged through the outlet again, thereby accurately determining whether the sensor strip has been used.

### DESCRIPTION OF DRAWINGS

FIG. 1 is a view illustrating a cartridge type bioinformation measurement device according to the present disclosure;
FIG. 2 is a view illustrating an example of a cartridge according to the present disclosure;
FIG. 3 is a view illustrating an example of a method of detecting whether a sensor strip has been used according to the present disclosure;
FIG. 4 is a view illustrating another example of a method of detecting whether a sensor strip has been used according to the present disclosure;
FIG. 5 is a functional block diagram illustrating an example of a management module according to the present disclosure;
FIG. 6 is a view illustrating an example of a measurement body according to the present disclosure; and
FIG. 7 illustrates an example in which information of a cartridge is displayed according to the present disclosure.

### MODE FOR INVENTION

The technical or scientific terms used herein are for the purpose of describing particular exemplary embodiments only and are not intended to be limiting of the present disclosure. In addition, the technical or scientific terms used herein should be understood as having the same meaning as commonly understood by a person skilled in the art to which the present disclosure relates, and should not be interpreted in an overly broad or narrow manner, unless expressly so defined herein. If any technical terms used herein fail in correctly representing the idea of the present disclosure, they should be substituted with technical terms correctly understandable by a person skilled in the art to which the present disclosure relates.

Descriptions of components in the singular form used herein are intended to include descriptions of components in the plural form, unless explicitly described to the contrary. It will be understood that the terms "comprise," "include," and any variations thereof used herein shall not be interpreted as essentially including all components or steps described herein, and are intended to cover non-exclusive inclusions unless explicitly described to the contrary.

In addition, the accompanying drawings shall be interpreted as being provided for a better understanding, while not being limitative, of the principle of the present disclosure.

Hereinafter, a cartridge type bioinformation measurement device according to the present disclosure will be described in more detail with reference to the accompanying drawings.

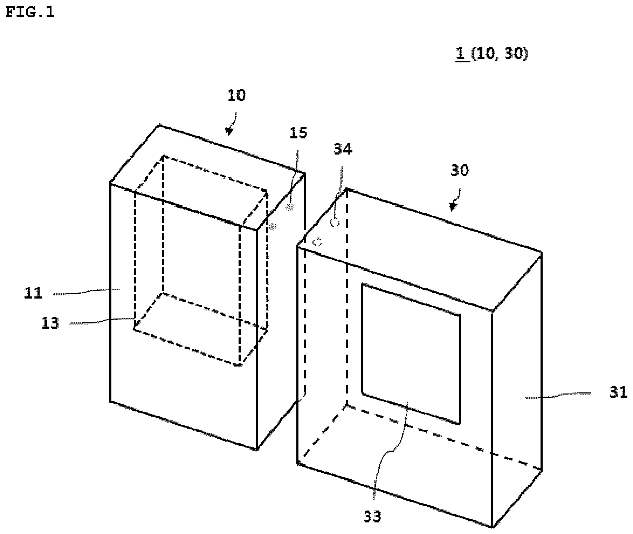

FIG. 1 is a view illustrating a cartridge type bioinformation measurement device according to the present disclosure.

Referring to FIG. 1, a cartridge type bioinformation measurement device 1 according to the present disclosure includes a cartridge 10 and a measurement body 30. The cartridge 10 includes a housing 11 which has an interior space 13 in which a plurality of sensor strips are stacked and stored. The cartridge 10 is typically coupled to the measurement body 30 to measure biometric information of a user using the sensor strips.

Terminal may be formed on one side of the housing 11 of the cartridge 10 and the other side of a housing 31 of the measurement body 30, which faces one side of the housing 11 when the cartridge 10 is coupled to the measurement body 30. Thus, the cartridge 10 or the measurement body 30 may determine whether the cartridge 10 and the measurement body 30 have been coupled to each other through contact between the terminal on one side and the terminal at the other side.

The cartridge 10 detects whether the sensor strip stored in the interior space 13 has been used and thus calculates and stores information on a number of the sensor strips remaining in the interior space 13 of the cartridge 10 according to the number of times of the detection of whether the sensor strip has been used. When or after the cartridge 10 is coupled to the measurement body 30, in a case in which the information on the number of the remaining sensor strips is requested from the measurement body 30, the stored information on the remaining number of the sensor strips is provided to the measurement body 30 and is output to a user through a display unit 33 of the measurement body 30. By using the information on the remaining number of the sensor strips output through the display unit 33, when the remaining number of the sensor strips remaining in the cartridge 10 is small, the user orders a new cartridge before all the sensor strips are used, thereby assisting bioinformation to be continuously measured.

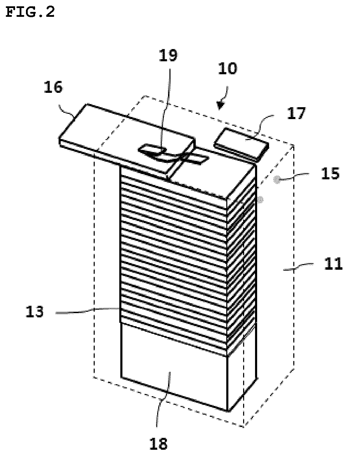

FIG. 2 is a view illustrating an example of a cartridge according to the present disclosure.

Describing in more detail with reference to FIG. 2, a plurality of sensor strips 16 are stacked and stored in an internal space 13 of a cartridge 10. When a user is to measure bioinformation, the user pushes one sensor strip of the stacked sensor strips to an outlet using a push button (not shown) formed in a measurement body and moves and fixes the sensor strip to a measurement position for measuring bioinformation of the user.

On the other hand, blood from the user is introduced to a front end of the sensor strip, and a plurality of electrodes is formed on an upper surface of the sensor strip. The electrodes formed on the upper surface of the sensor strip are connected to a measurement terminal 19 at the measurement position.

Here, the measurement terminal 19 is connected to a measurement body 30. The measurement body 30 measures the bioinformation of the user using a numerical value received through the measurement terminal 19. Preferably, a production lot number of a sensor strip is stored in a management module 17 of the cartridge 10. The measurement body 30 may accurately measure the bioinformation of the user using the production lot number. That is, the sensor strips may deviate from product to product. The sensor strips may deviate by the production date or production lot number. A numerical value measured using the sensor strip is corrected using a correction reference corresponding to the production lot number of the sensor strip.

After the measurement of the bioinformation is completed, the sensor strip disposed at the measurement position is discharged through the outlet by the push button. The management module 17 detects whether a sensor strip has been used by determining whether the sensor strip has been disposed at the measurement position from the interior space in order to measure the bioinformation or determining whether the sensor strip has been discharged through the outlet from the measurement position after the measurement has been completed. The measurement module 17 counts the number of sensor strips remaining in the interior space of the cartridge based on a detection result and stores and manages information on a remaining number of the sensor strips.

Here, a spring unit 18 is provided at a lower stage of the stacked and stored sensor strips to move the sensor strips to an upper stage. Accordingly, the sensor strips stacked and stored in the interior space are sequentially moved to the upper stage and are discharged through the outlet.

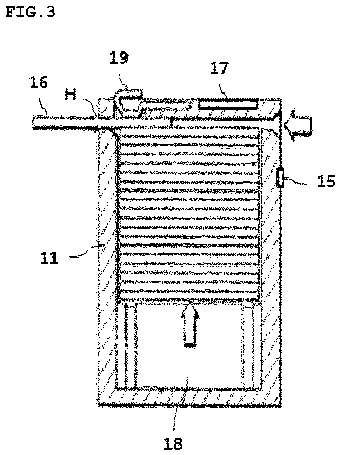

FIG. 3 is a view illustrating an example of a method of detecting whether a sensor strip has been used according to the present disclosure.

As illustrated in FIG. 3, a sensor strip 16 located at an upper stage of sensor strips stacked and stored in an interior space of a cartridge 10 is moved to an outlet H by a repulsive force generated by a push button and is disposed at a measurement position. When the sensor strip 16 is moved from a stack position to the measurement position, a terminal formed on an upper surface of the sensor strip comes into contact with a measurement terminal 19. It is detected whether the sensor strip has been used by determining that the sensor strip has been used whenever the terminal of the sensor strip and the measurement terminal 19 come into contact each other. A management module 17 calculates the final remaining number by deducting one from a number of the remaining sensor strips based on whether the sensor strip has been used. Thus, the management module 17 stores and manages information on the final remaining number of the sensor strips.

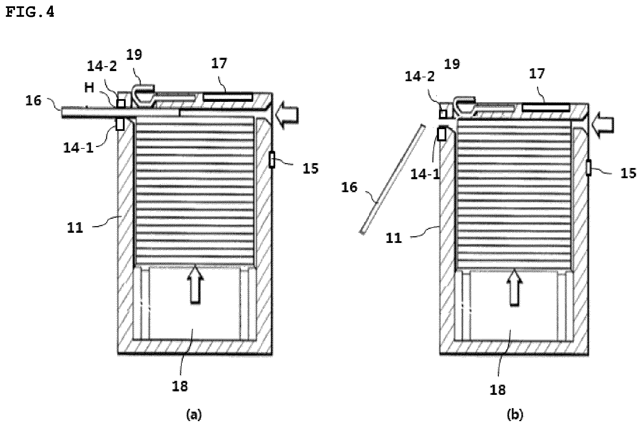

FIG. 4 is a view illustrating another example of a method of detecting whether a sensor strip has been used according to the present disclosure.

As illustrated in FIG. 4 (a), a sensor strip 16 located at an upper stage of sensor strips stacked and stored in an interior space of a cartridge is moved to an outlet H by a repulsive force generated by a push button and is disposed at a measurement position. An optical sensor 14 includes a light-emitting portion 14-1 and a light-receiving portion which are disposed across the outlet H so as to be opposite to each other. The light-emitting portion 14-1 is disposed at a lower end of the outlet H, and the light-receiving portion 14-2 is disposed at an upper end of the outlet H. A position of the light-emitting portion 14-1 and a position of the light-receiving portion 14-2 may be changed according to a field to which the present disclosure is applied, and this is within the scope of the present disclosure. Therefore, before the sensor strip 16 is moved to the measurement position, the light-receiving portion 14-2 may receive light emitted from the light-emitting portion 14-1, and when the sensor strip 16 is moved to the measurement position, the light-receiving portion 14-2 may not receive the light emitted from the light-emitting portion 14-1. When the light-receiver 14-2 may not receive the light emitted from the light-emitting unit 14-1, it is determined that the sensor strip has been used, and thus, it is detected whether the sensor strip has been used.

More preferably, as illustrated in FIG. 4 (b), after measurement of bioinformation is completed, the sensor strip 16 at the measurement position is pushed to the outlet H by the push button, the light-receiving portion 14-2 receives the light emitted from the light-emitting portion 14-1 again. As described above, one cycle including a step of receiving light, a step of not receiving light, and a step of receiving light again in this order is set. When one cycle has been performed, it may be detected that one sensor strip has been used. As described above, since it is detected whether the sensor strip has been used by using one cycle, it is possible to prevent an error in detecting whether the sensor strip has been used, caused by a malfunction of an optical sensor, and it is possible to accurately detect whether the sensor strip has been used.

A management module 17 calculates the final remaining number of sensor strips by deducting one from a number of the remaining sensor strips based on whether the sensor strip has been used. Thus, the management module 17 stores and manages information on the final remaining number of the sensor strips.

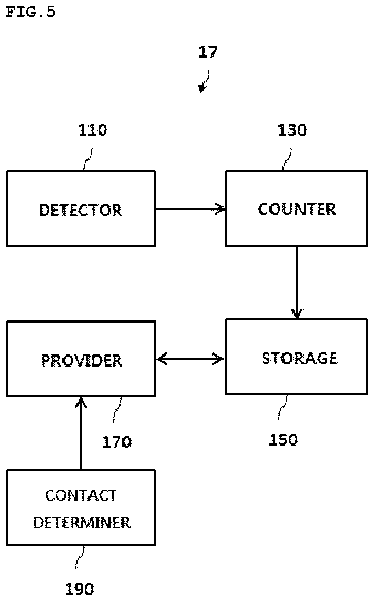

FIG. 5 is a functional block diagram illustrating an example of a management module according to the present disclosure.

Describing in more detail with reference to FIG. 5, a detector 110 determines whether a sensor strip has been used by detecting whether the sensor strip stacked and stored in an interior space has been moved to a measurement position and has been exposed and disposed at the measurement position or detecting whether the sensor strip has been moved from the measurement position and has been discharged through an outlet after measurement of bioinformation has been completed.

When it is determined that the sensor strip has been used based on whether the sensor strip has been used, a counter 130 calculates a final remaining number of the sensor strips currently remaining in the interior space by deducting one from a number of the sensor strips remaining in a storage 150. Thus, information on the counted final remaining number is stored and managed in the storage 150.

On the other hand, a contact determiner 190 determines whether a cartridge and a measurement body have been coupled by detecting whether one side terminal formed in the cartridge and the other side terminal formed in the measurement body have come into contact with each other. When the cartridge and the measurement body have been coupled, the contact determiner 190 generates and supplies a coupling completion signal to a provider 170. When the provider 170 receives the coupling completion signal, the provider 170 provides the information on the final remaining number stored in the storage 150 to the measurement body.

Preferably, when the provider 170 receives a user command for requesting the information on the final remaining number from the measurement body, the provider 170 may extract the information on the final remaining number stored in the storage 150 and may provide the extracted information to the measurement body, and this is within the scope of the present disclosure.

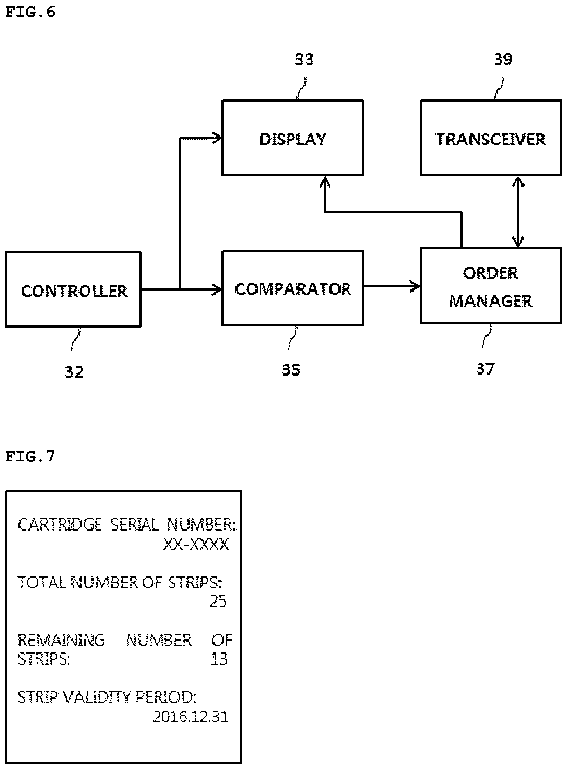

Preferably, the provider 170 monitors whether the final remaining number of the sensor strips stored in the storage 150 has been changed. Whenever the final remaining number of the sensor strips has been changed, the provider 170 provides the information on the final remaining number of the sensor strips to the measurement body. As illustrated in FIG. 7, whenever the final remaining number of the sensor strips has been changed, the measurement body displays the final remaining number of the sensor strips to a user. That is, whenever the user measures bioinformation using the sensor strip, information on the remaining number of the sensor strips remaining in the cartridge may be provided to the user, and thus, whenever bioinformation to be used is measured, the user may confirm the information on the remaining number of the sensor strips.

More Preferably, the provider 170 may provide information on the validity period of the cartridge stored in the storage 150 to the measurement body together with the information on the remaining number of the sensor strips, and the measurement body may display concurrently display the information on the remaining number of the sensor strips and the validity period of the sensor strip. Accordingly, the user may check a validity period of usable strips as well as the information on the information on the remaining number of the sensor strips.

FIG. 6 is a view illustrating an example of a measurement body according to the present disclosure.

Describing in more detail with reference to FIG. 6, when a controller 32 receives information on a final remaining number of sensor strips from a cartridge, the controller 32 displays the information on the final remaining number of the sensor strips on a display unit 33 to output the information on the final remaining number to a user.

On the other hand, the controller 32 provides the information on the final remaining number of the sensor strips to a comparator 35, and the comparator 35 compares the final remaining number of the sensor strips with a preset critical remaining number to compare whether the final remaining number of the sensor strips is lower than the critical remaining number.

When the final remaining number of the sensor strips is lower than the critical remaining number, the comparator 35 generates and provides an alarm message to an order manager 37. When the order manager 37 receives the alarm message, the order manager 37 generates an order message requesting an order for a new cartridge and performs a control to transmit the order message to a set user terminal through a transceiver 39. Preferably, the order manager 37 stores information on an address, a contact address, or the like of a user. The order manager 37 generates an inquire message for inquiring whether to requests the order for the new cartridge to the address or the contact address of the user and outputs the generated inquire message to the user through a display unit 33. When a confirmation message is received from the user in response to the inquiry message, the order manager 37 may generate an order message for requesting the order for the new cartridge to the stored address or contact address.

More preferably, the user may change the address or contact address of the user of the inquiry message or change an order number of the new cartridge through a user interface (not shown) and may change an order message according to the changed address or contact address of the user or the changed order number.

Here, the transceiver 39 is a device capable of communicating with a smartphone set by the user. For example, the transceiver 39 may communicate with the smartphone through a Bluetooth transmission.

When the transceiver 39 receives an order completion message from the smartphone, the transceiver 39 provides the order completion message to the order manager 37, and the order manager 37 displays the order completion message on the display unit to output the order completion message to the user.

Preferably, an input button (not shown) is separately provided on one side of the measurement body 30 to request information on a remaining number of the sensor strips remaining in the cartridge. When a user command for requesting the information on the remaining number of the sensor strips is input though the input button, the controller 32 may request the information on the remaining number of the sensor strips remaining in the cartridge to the provider 170 and may display the information on the remaining number of the sensor strips on the display unit 33. Therefore, the user may quickly and easily confirm the information on the remaining number of the sensor strips remaining in the cartridge through the input button.

The embodiments of the present disclosure may be written as computer programs and be implemented in general-use digital computers that execute the programs using a computer readable recording medium.

Examples of the computer readable recording medium include magnetic storage media (for example, ROMs, floppy disks, and hard disks), optical recording media (for example, CD-ROMs and DVDs), and storage media such as carrier waves (for example, transmission through the Internet).

While the present disclosure has been described with reference to certain exemplary embodiments shown in the drawings, these embodiments are illustrative only. Rather, it will be understood by a person skilled in the art that various modifications and equivalent other embodiments may be made therefrom. Therefore, the true scope of the present disclosure shall be defined by the appended claims.

## Claims

1. A cartridge type bioinformation measurement device comprising:
a cartridge (10) having an interior space (13) in which a plurality of strips for measuring bioinformation are stacked; and
a measurement body (30) on which the cartridge is detachably mounted, the measurement body being configured to measure the bioinformation through the strip exposed from the cartridge, **characterized in that**:
terminals are formed on one side of the cartridge (10) and the other side of the measurement body (30) facing the one side and come into contact with each other when the cartridge (10) is mounted on the measurement body (30), and a management module (17) is disposed in the cartridge to count and store a remaining number of the strips and provide information on the remaining number of the strips to the measurement body through the terminals,
wherein the management module (17) comprises:
a counter (130) configured to count the remaining number of the strips remaining in the interior space (13) based on whether the strip has been used;
a storage (150) configured to store the remaining number of the strips; and
a provider (170) configured to provide the information on the remaining number of the strips to the measurement body (30) through the terminals wherein the management module (17) further comprises a detector (110) configured to detect whether the strip has been used by determining whether the strip has been exposed from the interior space in order to measure bioinformation or determining whether the strip has been discharged from an exposure mounting position after the bioinformation has been measured, and wherein the detector (110) is an optical sensor (14) including a light-emitting portion (14-1) and a light-receiving portion (14-2) which are disposed across outlet (H) so as to be opposite to each other, and the detector (110) determines that the sensor strip has been used when the light-receiving portion (14-2) does not receive light emitted from the light-emitting portion (14-1).

2. The cartridge type bioinformation measurement device according to claim 1, wherein the detector (110) is the optical sensor (14) configured to detect whether the strip is exposed or to detect whether the strip is discharged from the exposure mounting position.

3. The cartridge type bioinformation measurement device of claims 1 or 2, wherein the measurement body (30) further comprises a display unit (33) configured to display the information on the remaining number of the strips received from the management module (17).

4. The cartridge type bioinformation measurement device according to claim 3, wherein the management module (17) is configured to determine whether the terminal formed on the one side of the cartridge (10) and the cartridge formed at the other side of the measurement body (30) come into contact with each other, and when the terminal on the one side and the other terminal at the other side come into contact with each other, the management module (17) is further configured to provide the information on the remaining number of the strips to the measurement body (30) and to display the information on the remaining number on the display unit (33).

5. The cartridge type bioinformation measurement device according to claim 4, wherein the measurement body (30) further comprises an order manager (37) configured to generate an order message for requesting an order for a new cartridge and to transmit the order message to a set user terminal.

6. The cartridge type bioinformation measurement device according to claim 5, wherein the measurement body (30) further comprises a comparator (35) configured to compare the remaining number of the sensor strips received from the management module with a preset critical remaining number, and when the remaining number of the sensor strips is lower than the critical remaining number, the order manager (37) is configured to generate the order message.

## Patentansprüche

1. Kassettenartige Vorrichtung zur Messung von biologischen Informationen, umfassend:
eine Kassette (10), die einen Innenraum (13) aufweist, in dem eine Vielzahl von Streifen zum Messen von biologischen Informationen gestapelt sind, und
einen Messkörper (30), an dem die Kassette abnehmbar montiert ist, wobei der Messkörper dazu konfiguriert ist, die biologischen Informationen über den aus der Kassette freigelegten Streifen zu messen, **dadurch gekennzeichnet, dass**:
auf einer Seite der Kassette (10) und der anderen Seite des Messkörpers (30), die der einen Seite zugewandt ist, Anschlüsse ausgebildet sind und bei der Montage der Kassette (10) auf dem Messkörper (30) miteinander in Kontakt kommen, und ein Verwaltungsmodul (17) in der Kassette angeordnet ist, um eine verbleibende Anzahl der Streifen zu zählen und zu speichern und dem Messkörper über die Anschlüsse Informationen über die verbleibende Anzahl der Streifen bereitzustellen,
wobei das Verwaltungsmodul (17) Folgendes umfasst:
einen Zähler (130), der dazu konfiguriert ist, die verbleibende Anzahl der in dem Innenraum (13) verbleibenden Streifen zu zählen, basierend darauf, ob der Streifen verwendet wurde,
einen Speicher (150), der dazu konfiguriert ist, die verbleibende Anzahl der Streifen zu speichern; und
einen Bereitsteller (170), der dazu konfiguriert ist, dem Messkörper (30) über die Anschlüsse die Informationen über die verbleibende Anzahl der Streifen bereitzustellen, wobei das Verwaltungsmodul (17) ferner einen Detektor (110) umfasst, der dazu konfiguriert ist, zu detektieren, ob der Streifen verwendet wurde, durch Bestimmen, ob der Streifen aus dem Innenraum freigelegt wurde, um biologische Informationen zu messen, oder Bestimmen, ob der Streifen aus einer Freilegungsmontageposition abgegeben wurde, nachdem die biologischen Informationen gemessen wurden, und wobei der Detektor (110) ein optischer Sensor (14) ist, der einen lichtemittierenden Abschnitt (14-1) und einen lichtempfangenden Abschnitt (14-2) enthält, die über dem Auslass (H) angeordnet sind, so dass sie einander gegenüberliegen, und der Detektor (110) bestimmt, dass der Sensorstreifen verwendet wurde, wenn der lichtempfangende Abschnitt (14-2) kein von dem lichtemittierenden Abschnitt (14-1) emittiertes Licht empfängt.

2. Kassettenartige Vorrichtung zur Messung von biologischen Informationen nach Anspruch 1, wobei der Detektor (110) der optische Sensor (14) ist, der dazu konfiguriert ist, zu detektieren, ob der Streifen freigelegt ist, oder zu detektieren, ob der Streifen aus der Freilegungsmontageposition abgegeben ist.

3. Kassettenartige Vorrichtung zur Messung von biologischen Informationen nach den Ansprüchen 1 oder 2, wobei der Messkörper (30) ferner eine Anzeigeeinheit (33) umfasst, die dazu konfiguriert ist, die von dem Verwaltungsmodul (17) empfangenen Informationen über die verbleibende Anzahl der Streifen anzuzeigen.

4. Kassettenartige Vorrichtung zur Messung von biologischen Informationen nach Anspruch 3, wobei das Verwaltungsmodul (17) dazu konfiguriert ist, zu bestimmen, ob der Anschluss, der auf der einen Seite der Kassette (10) ausgebildet ist, und die Kassette, die auf der anderen Seite des Messkörpers (30) ausgebildet ist, miteinander in Kontakt kommen, und wobei, wenn der Anschluss auf der einen Seite und der andere Anschluss auf der anderen Seite miteinander in Kontakt kommen, das Verwaltungsmodul (17) ferner dazu konfiguriert ist, die Informationen über die verbleibende Anzahl der Streifen dem Messkörper (30) bereitzustellen und die Informationen über die verbleibende Anzahl auf der Anzeigeeinheit (33) anzuzeigen.

5. Kassettenartige Vorrichtung zur Messung von biologischen Informationen nach Anspruch 4, wobei der Messkörper (30) ferner einen Auftragsverwalter (37) umfasst, der dazu konfiguriert ist, eine Auftragsnachricht zum Anfragen eines Auftrags für eine neue Kassette zu erzeugen und die Auftragsnachricht an einen eingestellten Benutzeranschluss zu übermitteln.

6. Kassettenartige Vorrichtung zur Messung von biologischen Informationen nach Anspruch 5, wobei der Messkörper (30) ferner einen Komparator (35) umfasst, der dazu konfiguriert ist, die verbleibende Anzahl der Sensorstreifen, die von dem Verwaltungsmodul empfangen wird, mit einer voreingestellten kritischen verbleibenden Anzahl zu vergleichen, und, wenn die verbleibende Anzahl der Sensorstreifen geringer als die kritische verbleibende Anzahl ist, der Auftragsverwalter (37) dazu konfiguriert ist, die Auftragsnachricht zu erzeugen.

## Revendications

1. Dispositif de mesure de bio-informations de type cartouche comprenant :
une cartouche (10) comportant un espace intérieur (13) dans lequel une pluralité de bandes destinées à mesurer les bio-informations sont empilées ; et
un corps de mesure (30) sur lequel la cartouche est montée de manière amovible, le corps de mesure étant conçu pour mesurer les bio-informations à travers la bande exposée à partir de la cartouche, **caractérisé en ce que** :
des bornes sont formées d'un côté de la cartouche (10) et de l'autre côté du corps de mesure (30) faisant face audit un côté et viennent en contact l'une avec l'autre lorsque la cartouche (10) est montée sur le corps de mesure (30), et un module de gestion (17) est disposé dans la cartouche pour compter et stocker le nombre restant de bandes et délivrer les informations sur le nombre restant de bandes au corps de mesure par l'intermédiaire des bornes,
ledit module de gestion (17) comprenant :
un compteur (130) conçu pour compter le nombre restant de bandes restant dans l'espace intérieur (13) selon que la bande ait été utilisée ;
un stockage (150) conçu pour stocker le nombre restant de bandes ; et
un dispositif fournisseur (170) conçu pour délivrer les informations sur le nombre restant de bandes au corps de mesure (30) par l'intermédiaire des bornes, ledit module de gestion (17) comprenant en outre un détecteur (110) conçu pour détecter si la bande a été utilisée en déterminant si la bande a été exposée depuis l'espace intérieur afin de mesurer les bio-informations ou en déterminant si la bande a été déchargée d'une position de montage d'exposition après la mesure des bio-informations, et ledit détecteur (110) étant un capteur optique (14) comprenant une partie émettrice de lumière (14-1) et une partie réceptrice de lumière (14-2) qui sont disposées à travers la sortie (H) de manière à être opposées l'une à l'autre, et ledit détecteur (110) déterminant que la bande de capteur a été utilisée lorsque la partie réceptrice de lumière (14-2) ne reçoit pas la lumière émise par la partie émettrice de lumière (14-1).

2. Dispositif de mesure de bio-informations de type cartouche selon la revendication 1, ledit détecteur (110) étant le capteur optique (14) conçu pour détecter si la bande est exposée ou pour détecter si la bande est déchargée de la position de montage d'exposition.

3. Dispositif de mesure de bio-informations de type cartouche selon la revendication 1 ou 2, ledit corps de mesure (30) comprenant en outre une unité d'affichage (33) conçue pour afficher les informations sur le nombre restant de bandes reçues du module de gestion (17).

4. Dispositif de mesure de bio-informations de type cartouche selon la revendication 3, ledit module de gestion (17) étant conçu pour déterminer si la borne formée sur ledit un côté de la cartouche (10) et la cartouche formée sur ledit autre côté du corps de mesure (30) entrent en contact l'une avec l'autre, et lorsque la borne sur ledit un côté et l'autre borne sur ledit autre côté entrent en contact l'une avec l'autre, ledit module de gestion (17) étant en outre conçu pour délivrer les informations sur le nombre restant de bandes au corps de mesure (30) et pour afficher les informations sur le nombre restant sur l'unité d'affichage (33).

5. Dispositif de mesure de bio-informations de type cartouche selon la revendication 4, ledit corps de mesure (30) comprenant en outre un gestionnaire de commande (37) conçu pour générer un message de commande afin de demander la commande d'une nouvelle cartouche et pour transmettre le message de commande à un terminal utilisateur défini.

6. Dispositif de mesure de bio-informations de type cartouche selon la revendication 5, ledit corps de mesure (30) comprenant en outre un comparateur (35) conçu pour comparer le nombre restant de bandes de détection reçues du module de gestion au nombre restant critique prédéfini, et lorsque le nombre restant de bandes de détection est inférieur au nombre restant critique, ledit gestionnaire de commande (37) étant conçu pour générer le message de commande.
